(19) Europäisches Patentamt
European Patent Office
Office européen des brevets

(11) Publication number: 0 232 435 A2

# (12) EUROPEAN PATENT APPLICATION

(21) Application number: **85309232.8**

(22) Date of filing: **18.12.85**

(51) Int. Cl.⁴: **C 07 K 7/06**
// A61K37/00

(43) Date of publication of application: **19.08.87**
**Bulletin 87/34**

(84) Designated Contracting States: **AT BE CH DE FR GB IT LI LU NL SE**

(71) Applicant: **SPOFA Spojené Podniky Pro Zdravotnickou Vyrobu, Husinecká 11 a, Prag 3 (CS)**

(72) Inventor: **Kasafirek, Evzen, CSc, No 27 Lomena, Praha 6 (CS)**
Inventor: **Cerny, Martin, No 7 Palackeho, Praha 1 (CS)**
Inventor: **Kocis, Petr, No 13 Kubelikova, Praha 3 (CS)**
Inventor: **Krepelka, Jiri, CSc, No 26 Madridska, Praha 10 (CS)**
Inventor: **Rovensky, Josef, MUDr CSc, No 27 Csl. Armady, Piestany (CS)**

(74) Representative: **Wotherspoon, Graham et al, FITZPATRICKS 4 West Regent Street, Glasgow G2 1RS Scotland (GB)**

## (54) Serum thymic factor peptide analogs and a process for the preparation thereof.

(57) Novel peptide analogs of the serum thymic factor are structurally modified, in comparison with the natural substance, both in their N-terminal and C-terminal parts and inside the amino-acid sequence. They correspond to the general formula I

A – Gly – Gly – Ser – Asn – B – C – NH – R (I)

in which A is pGlu, Gln, Ala-Lys-Ser, pGlu-Ala-Lys-Ser or Gln-Ala-Lys-Ser, B and C are Gly, Phe, Leu, Ala or a direct bond and R is H, an alkyl with 1 to 6 carbon atoms or a 2-phenylethyl.

Dependently on their chemical structure, the subject thymic factor analogs, possess either agonistic (immunostimulative) or antagonistic (immunosuppressory) properties.

EP 0 232 435 A2

# SERUM THYMIC FACTOR PEPTIDE ANALOGS AND A PROCESS FOR THE PREPARATION THEREOF

The invention relates to serum thymic factor peptide analogs and to a process for the preparation thereof.

Thymic agonists, either the natural hormone isolated from thymus or its synthetic analogs, have recently been adopted in the treatment of immunodeficiency for increasing the patients' immunity response, eg in synthetic diseases lupus erythematodes, myasthenia gravis, infantile asthma, and also in certain neoplastic (neoplasmic) diseases.

The search for serum thymic factor peptide analogs has heretofore focused on the modification of the natural serum thymic factor molecule either inside the amino-acid sequence (pGlu-Ala-Lys-Ser-Gln-$Gly_2$-Ser-Asn) or at its N-terminus (ie, the left-hand end). On the contrary, not a single attempt of modification or prolongation at the opposite C-terminus (the right-hand end) of the sequence has been reported.

An object of this invention is to provide further alternative serum thymic factor peptide analogs.

According to this invention there are provided serum thymic factor peptide analogs of the general formula I

A - Gly - Ser - Asn - B - C - NH - R     (I)

in which A is pGlu, Gln, Ala-Lys-Ser, pGlu-Ala-Lys-Ser or Gln-Ala-Lys-Ser, B and C are Gly, Phe, Leu, Ala or a direct bond and R is H, an alkyl with 1 to 6 carbon atoms or a 2-phenylethyl group.

As is evident from the general formula I in comparison with the above structure of the natural substance, the compounds of the present invention, besides optional shortening at the N-terminus, comprise a C-terminal augmentation predominantly by an additional amino-acid residue or residues bearing an amide moiety. They surprisingly proved to possess, depending on their particular structure, a remarkable immunomodulating activity, either in the agonistic (stimulatory) or antagonistic (moderative or even suppressory) sense. Their in-vitro

potency, as assessed by the standard E-rosette test (Clin. Exp. Immunol. 47, 183, 1982) is comparable to that of natural thymus hormones, eg serum thymic factor and purified thymosine fraction V, and in several typical examples it is significantly superior. The respective numerical assay data (positive for immunostimulation and negative for immunosuppression) are summarized in the following table.

| Thymic factor structure or name | Rosette recombination differential count, % | Please note |
|---|---|---|
| p-Glu - D - Gln - E (natural) | 9.20 | D = Ala - Lys - Ser |
| Thymosine fraction V | 3.80 | |
| D - Gln - E | 3.18 | E = $Gly_2$- Ser - Asn |
| D - Gln - E - $GlyNH_2$ | 12.75 | |
| D - Gln - E - $PheNH_2$ | 16.09 | |
| p-Glu - D - Gln - E - $GlyNH_2$ | 6.31 | |
| p-Glu - D - Gln - E - $PheNH_2$ | 7.54 | |
| Gln - D - Gln - E | -1.64 | |
| Gln - D - Gln - E - $PheNH_2$ | -1.52 | |
| D - Gln - E - PheNHR | -6.0 | R = 2-Ph-ethyl |
| p-Glu ----------- E - $GlyNH_2$ | 6.35 | |
| p-Glu ----------- E - $PheNH_2$ | 2.89 | |
| p-Glu ----------- E - PheNHR | -13.0 | R = 2-Ph-ethyl |
| Gln ----------- E - $PheNH_2$ | -22.9 | |

The heretofore performed structural modifications at the N-terminus of the amino-acid sequence of the serum thymic factor indicated the nonsignificance of the pyroglutamyl or glutaminyl residues in position 1, ie, their presence had only a minor effect on the activity. (Citace G. TSUKAMOTO et al : J. Protein Chem. 1,305 (1982)). Contrary to this, in the subject serum thymic factor peptide analogs that comprise an additional C-terminal amino-acid residue terminated with an amide or substituted amide group,

the chemical nature of their N-terminal position is critical for the degree and even for the sense of activity.

The serum thymic factor peptide analogs of the invention can be prepared by common preparative methods generally employed in the synthesis of peptides, eg by combining appropriate peptidic fragments (fragment condensation) or by successive step-wise build-up of the desired amino-acid sequence. The process can be conducted either in solution or on a solid carrier, optionally under enzymatic catalysis, and any known protective groups and condensation agents known in the art can principally be used.

The subject peptide compounds of the general formula I can advantageously be prepared according to the invention by reacting a peptide compound of the general formula II

Gly - Gly - Ser - Asn - B - C - NH - R (II)

in which B, C and R have the same meanings as in formula I, with a compound of the general formula III

X - A (Y) (III)

in which A has the same meaning as in formula I and X and Y are protective groups for protecting alpha or omega amino groups, with the proviso that X and Y can also be H atoms when A is pGlu, to yield a peptide compound of the general formula IV

X - A (Y) - Gly - Ser - Asn - B - C - NH - R (IV)

in which A, B, C and R have the same meanings as in formula I and X and Y have the same meanings as in formula III, with an optional subsequent step involving successive or simultaneous removal of the protective groups by per se known techniques.

Those of the subject compounds of the general formula I in which A stands for a pyroglutamyl residue and B, C and R have the aforementioned meaning can advantageously be obtained by reacting the respective compound of the general formula II with unprotected pyroglutamic acid.

Starting materials required for the preparation are

known substances that are available by known methods described in the pertinent literature.

Further particulars of the preparative procedures are illustrated by the following non-limitative examples.

Explanatory notes on the experimental procedures.

Melting temperatures were determined on Kofler apparatus and are not corrected. Analytical samples were dried under reduced pressure at 70 kPa over phosphorus pentoxide at room temperature (for compounds melting below 115°C) or at 105°C (for compounds melting above 115°C). Optical rotations were measured at 20°C and the Na D line wavelength on Perkin-Elmer 141 polarimeter, the content of water was determined on Aquatest II Photovolta instrument.

Chromatographic separations were performed on thin layer of silica gel (Kieselgel G, Merck) in the following solvent systems:

$S_1$ : 1-butanol - acetic acid - water 4:1:1
$S_2$ : 1-butanol - acetic acid - pyridine - water 15:3:10:6
$S_3$ : 2-propanol - water 2:1

The detection of free amino acids and peptides was effected with ninhydrin, the detection of protected peptides was carried out by the standard chlorination technique with the use of 2-tolidine.

Evaporation of solutions was carried out under reduced pressure on a rotary vacuum evaporator. Unless otherwise stated, the hydrogenations were conducted at atmospheric pressure and ambient temperature. Most of the crystallizations were carried out by the binary solvent technique: the material was dissolved in the first solvent at its boiling temperature and the other solvent indicated was added to induce the crystallization on cooling. Analytical samples were recrystallized, unless otherwise stated, in a similar manner from the preceding solvent system.

The composition of all prepared compounds including

new intermediates referred to herein below was verified by elemental analysis, the composition of presumed solvates also by the determination of respective solvent.

Example 1

a) benzyloxycarbonylasparaginyl-phenylalanine methyl ester

A solution of benzyloxycarbonylasparagine (13.3 g; 50 mmoles), N-ethylpiperidine (7.0 ml) and pyridine (5.0 ml) in dichloromethane (100 ml) cooled to -20°C was treated under constant stirring with pivaloylchloride (6.3 ml); the mixture was then stirred and cooled (0°C) for 8 minutes. After that, within 2 to 3 minutes, a solution of phenylalanine methyl ester, liberated from its hydrochloride (10.7 g; 50 mmoles) by the addition of N-ethylpiperidine (7.0 ml), in dichlormethane (100 ml) was added. The reaction mixture was stirred for an additional 2 hours at room temperature, the precipitated crystalline substance was collected on filter and triturated successibely with 1 M hydrochloric acid, water, 5% aqueous sodium hydrogencarbonate solution and water. Crystallization from methanol yielded 14.5 g (68%) of the title product melting at 196-197°C. An analytical sample was crystallized from dimethylformamide and 2-propanol, the m.p. remained unchanged. Chromatography: $R_f$ 0.71/$S_1$, 0.75/$S_2$. Optical rotation $[\alpha]_D^{20}$ -2.0° (c 0.2; dimethylformamide). The elemental composition corresponded to the summary formula $C_{22}H_{25}N_3O_6$.

b) Asparaginyl-phenylalanine methyl ester hydrobromide

The preceding methyl ester (4.3; 10 mmoles) dissolved in glacial acetic acid (7.0 ml) was treated with a 38% hydrogen bromide solution in acetic acid (10 ml) and the mixture was allowed to stand at room temperature for 1 hour. The product was precipitated by the addition of ether, separated, washed with ether and dried in a desiccator over sodium hydroxide and phosphorus pentoxide to give 3.6 g (97%) of the title substance, homogeneous electrophoretically

and chromatographically. $R_f$ 0.37/$S_1$, 0.70/$S_2$.

c) Benzyloxycarbonylseryl-asparaginyl-phenylalanine methyl ester

Benzyloxycarbonylserine (8.4 g; 35 mmoles), N-ethylpiperidine (4.9 ml) and pyridine (3.5 ml) in dichloromethane (100 ml) cooled to -20°C was treated under constant stirring with pivaloylchloride (4.4 ml); the mixture was stirred and left to stand whilst being cooled (0°C) for 8 minutes. After that, within 2-3 minutes, a solution of asparaginylphenylalanine methyl ester, liberated from its hydrobromide (13.1 g; 35 mmoles) by the addition of N-ethylpiperidine (4.9 ml), in dichloromethane (100 ml) was added. The reaction mixture was stirred for 2 hours at room temperature and evaporated. The noncrystalline residue was dissolved in ethyl acetate and the solution was successively washed with 1 M hydrochloric acid, water, 5% sodium hydrogencarbonate and water, dried over anhydrous sodium sulfate, evaporated and the residue was crystallized from methanol. The yield was 9.1 g (51%), m.p. 180-183°C. A sample was recrystallized from the same solvent and had m.p. 184-186°C. $R_f$ 0.71/$S_1$, 0.82/$S_2$. $[\alpha]_D^{20}$ -13.8° (c 0.2;methanol). Composition: $C_{25}H_{30}N_4O_8$.

d) Benzyloxycarbonylseryl-asparaginyl-phenylalanine amide

A precooled solution of the preceding ester (5.2 g; 10 mmoles) in 98% methanol (130 ml) was treated with liquid ammonia (approx 33 g) and the reaction mixture was allowed to stand at room temperature for 70 hours. The precipitated crystalline product was separated and washed with methanol to yield 3.9 g (78%) of the product melting at 218-221°C. A sample was crystallized from dimethylformamide - methanol (1:1) by the addition of ether and had m.p. 222-224°C. $R_f$ 0.64/$S_1$, 0.71/$S_2$. $[\alpha]_D^{20}$ -15.9° (c 0.2; dimethylformamide). Composition: $C_{24}H_{29}N_5O_7$.

e) Seryl-asparaginyl-phenylalanine amide

A solution of the preceding amide (5.0 g; 10 mmoles) and acetic acid (0.1 ml) in 70% methanol (400 ml) was admixed with Pd black (approx 50 mg) and hydrogenated for 3 hours. After removal of the catalyst the solution was evaporated and the solid residue was crystallized from 95% methanol - ether to give 3.6 g (96%) of the product, m.p. 169-172°C. A sample was recrystallized, the m.p. remained unchanged. $R_f$ 0.23/$S_1$, 0.57/$S_2$. $[\alpha]_D^{20}$ -19.3° (c.0.2; methanol). Composition: $C_{16}H_{23}N_5O_5$.1/2 $H_2O$.

f) Benzyloxycarbonylglutaminyl-glycyl-glycine methyl ester

A suspension of glycyl-glycine methyl ester hydrochloride (1.9 g; 10 mmoles) in a dimethylformamide - dichloromethane (1:2) mixture (30 ml) was admixed with N-ethylpiperidine (1.4 ml) and stirred for 2 hours at room temperature. After that, benzyloxycarbonylglutamine pentachlorphenyl ester (5.3 g; 10 mmoles) was added and the reaction mixture was stirred at room temperature still for 4 hours. After 8 hours of standing the precipitated crystalline product was separated and crystallized from methanol - dichloromethane. Yield 2.5 g (61%), m.p. 129-134°C, after additional crystallization from methanol 132-134°C. $R_f$ 0.57/$S_1$, 0.78/$S_2$. $[\alpha]_D^{20}$ -8.5° (c 0.2; 50% acetic acid). Composition: $C_{18}H_{24}N_4O_7$. 1/2 $H_2O$.

g) Benzyloxycarbonylglutaminyl-glycyl-glycine hydrazide

To the preceding ester (8.3 g; 20 mmoles) dissolved in methanol (100 ml) there was added 100% hydrazine hydrate (4.7 ml). After 48 hours of standing at room temperature the crystalline deposit was separated, washed with methanol and crystallized from 80% ethanol to yield 6.1 g (74%) of the product melting at 169-171°C. $R_f$ 0.30/$S_1$, 0.70/$S_2$. $[\alpha]_D^{20}$ -9.1° (c 0.2; methanol). Composition: $C_{17}H_{24}N_6O_6$.

h) Benzyloxycarbonylglutaminyl-glycyl-glycyl-seryl-asparaginyl-phenylalanine amide

A solution of the preceding hydrazide (4.1 g; 10 mmoles) and concentrated hydrochloric acid (4.0 ml) in dimethylformamide (50 ml) cooled to -20°C was admixed with 3.6 M sodium nitrite solution (2.8 ml). The reaction mixture was stirred and cooled (-20°C) for 10 minutes, then adjusted by the addition of N-ethylpiperidine to pH 6.9 and a soluiton of seryl-asparaginyl-phenylalanine amide (3.7 g; 10 mmoles) in dimethylformamide (50 ml) was added. After 12 hours of standing at 0°C, the solution was evaporated and the solid residue was triturated successively with 1 M hydrochloric acid, water, 5% sodium hydrogencarbonate solution and water and crystallized from 1-butanol - ethyl acetate. Yield 5.6 g (74%), m.p. 225-227°C. A sample was recrystallized similarly and had m.p. 226-229°C. $R_f$ 0.30/$S_1$, 0.58/$S_2$. $[\alpha]_D^{20}$-12.1° (c 0.2; dimethylformamide). Amino-acid analysis: Glu 1.00, Gly 2.02, Ser 0.76, Asp 0.94, Phe 0.92. Composition: $C_{33}H_{43}N_9O_{11}$. $H_2O$.

i) Glutaminyl-glycyl-glycyl-seryl-asparaginyl-phenylalanine amide

The preceding amide (2.2 g; 3 mmoles) was hydrogenated in dimethylformamide - methanol (1:2; 100 ml) acidified with acetic acid (0.1 ml) over Pd black (approx 25 mg) for 6 hours. After separation of the catalyst the filtrate was evaporated and the solid residue was crystallized from 95% methanol by the addition of ether. Yield 1.7 g (86%), m.p. 202-204°C, after recrystallization 209-212°C. $R_f$ 0.05/$S_1$, 0.42/$S_2$. $[\alpha]_D^{20}$ -21.9° (c 0.2; 50% methanol). Composition: $C_{25}H_{37}N_9O_9$. $3H_2O$.

Example 2

a) Alanyl-Nω-benzyloxycarbonyllysyl-serine methyl ester hydrochloride

A solution of tert-butyloxycarbonylalanyl-Nω-benzyloxycarbonyllysyl-serine methyl ester (3.4 g; 6 mmoles) in glacial acetic acid (12 ml) was treated with 1 M hydrogen chloride solution in the same solvent (12 ml). The reaction

mixture was allowed to stand at room temperature for 1 hour and the product was precipitated with ether, separated and dried in a desiccator over sodium hydroxide and phosphorus pentoxide to yield 2.7 g (93%) of the title compound with $R_f$ 0.35/$S_1$, 0.62/$S_2$.

b) Pyroglutamyl-alanyl-Nω-benzyloxycarbonyllysyl-serine methyl ester

A solution of pyroglutamic acid (0.9 g; 7 mmoles), N-hydroxybenztriazole (0.95 g) and N,N′-dicyclohexylcarbodiimide (1.6 g) in dimethylformamide (50 ml) was stirred and cooled (-10°C) for 10 minutes. The reaction mixture was treated with a precooled (-10°C) solution of alanyl-N-benzyloxycarbonyllysyl-serine methyl ester, liberated from the preceding hydrochloride (3.4 g; 7 mmoles) by the addition of N-ethylpiperidine (1.0 ml), in dimethylformamide (50 ml). The reaction mixture was stirred for 2 hours at 0°C and then allowed to stand for 8 hours at room temperature. The N,N′-dicyclohexylurea precipitate was filtered off, the filtrate was evaporated, the noncrystalline residue was dissolved in ethyl acetate, washed successively with 1% aqueous citric acid, water, 5% sodium hydrogencarbonate and water, dried over anhydrous sodium sulfate, evaporated and crystallized from 2-propanol. The yield was 2.4 g (59%), m.p. 120-122°C. After recrystallization the product melted at 135-137°C and had $R_f$ 0.53/$S_1$, 0.83/$S_2$. $[\alpha]_D^{20}$ -24.4° (c 0.2; dimethylformamide). Amino-acid analysis: Glu 0.85, Lys 1.00, Ala 1.06, Ser 0.82. Composition: $C_{26}H_{37}N_5O_9 \cdot H_2O$.

c) Pyroglutamyl-alanyl-N -benzyloxycarbonyllysyl-serine hydrazide

The preceding ester (2.9 g; 5 mmoles) was dissolved in dimethylformamide - methanol (1:1; 40 ml) and 100% hydrazine hydrate (3.0 ml) was added. After 8 hours of standing at room temperature the precipitated crystalline product was collected on filter and washed with methanol.

The yield was 2.3 g (79%), m.p. 212-214°C, after recrystallization from 80% methanol 217-218°C. $R_f$ 0.54/$S_1$, 0.73/$S_2$. $[\alpha]_D^{20}$ ]48.9° (c 0.2; 80% methanol). Composition: $C_{25}H_{37}N_7O_8 \cdot H_2O$.

d) Pyroglutamyl-alanyl-Nω-benzyloxycarbonyllysyl-seryl-glutaminyl-glycyl-glycyl-seryl-asparaginyl-phenylalanine amide

A solution of the preceding hydrazide (0.95 g; 1.63 mmoles) and concentrated hydrochloric acid (0.6 ml) in dimethylformamide (20 ml) precooled to -20°C was admixed with a 3.6 M sodium nitrate solution (0.5 ml). After adjusting the mixture by the addition of N-ethylpiperidine to pH 6.9 a solution of glutaminyl-glycyl-glycyl-seryl-asparaginyl-phenylalanine amide (0.92 g; 1.51 mmoles) in dimethylformamide (40 ml) was added. After 12 hours standing at 0°C the reaction mixture was evaporated and noncrystalline residue was mixed with a saturated sodium chloride solution (2.0 ml); after standing for another hour at the same temperature the crystalline product was separated, washed and crystallized from butanol saturated with water at 20°C by the addition of ethyl acetate and recrystallized from 80% aqueous methanol - ethyl acetate. Yield 0.58 g (33%), m.p. 248-252°C, after recrystallization 259-261°C. $R_f$ 0.08/$S_1$, 0.42/$S_2$. $[\alpha]_D^{20}$ -48.2° (c 0.1; 50% aqueous acetic acid). Amino analysis: Glu 1.90, Ala 1.18, Lys 1.06, Ser 1.79, Gly 1.95, Asp 1.05, Phe 1.00. Composition: $C_{50}H_{70}N_{14}O_{17} \cdot 2H_2O$.

e) Pyroglutamyl-alanyl-lysyl-seryl-glutamyl-glycyl-glycyl-seryl-asparaginyl-phenylalanine amide

The preciding amide (300 mg; 0.26 mmole) was hydrogenated over Pd black (approx 20 mg) in 10% aqueous acetic acid (100 ml) at atmospheric pressure and room temperature for 3 hours. After separation of the catalyst the solution was evaporated, the residue was dissolved in 0.2 M acetic acid (5.0 ml) and purified by gel filtration on Sephadex

(Trade Mark) G 15 (column size 1.8 x 90 cm); homogeneous fractions were concentrated and freeze-dried to give 200 mg (64%) of the chromatographically uniform product. $R_f$ 0.02/$S_1$, 0.25/$S_2$. $[\alpha]_D^{20}$ -67.9° (c 0.1; 0.1 M acetic acid). Amino-acid analysis: Glu 1.94, Ala 1.18, Lys 1.10, Ser 1.78, Gly 2.03, Asp 1.04, Phe 1.00. Composition: $C_{42}H_{64}N_{17}O_{15}$ 2 AcOH . 4 $H_2O$.

Example 2A

Pyroglutamyl-glycyl-glycyl-seryl-asparaginyl-phenylalanine amide

Benzyloxycarbonylglutaminyl-glycyl-glycyl-glycyl-seryl-asparaginyl-phenylalanine amide (4.0 g; 5.26 mmoles) in 10% acetic acid (100 ml) was hydrogenated over Pd black (approx 25 mg) at 40°C under atmospheric pressure for 3 hours. After filtering off the catalyst the solution was evaporated, the remaining crude product was dissolved in 0.2 M acetic acid (5 ml) and purified by gel filtration on Sephadex G 15; homogeneous fractions were combined, concentrated and freeze-dried. The yield was 2.5 g (77%) m.p. 218 -221°C. $[\alpha]_D^{20}$ -35.7° (c 0.1, 1 M acetic acid). The substance was chromatographically uniform (ninhydrin test was negative). $R_f$ 0.15/$S_1$, 0.56/$S_2$. Amino-acid analysis: Glu 1.00, Gly 2.00, Ser 0.87, Asp 1.04, Phe 0.97. Composition: $C_{24}H_{33}N_8O_9$.

Example 3

a) tert-Butyloxycarbonylalanyl-N -benzyloxycarbonyl-lysyl-serine

A solution of tert-butyloxycarbonylalanyl-N -benzyloxy-carbonyllysyl-serine methyl ester (2.0 g, 3.6 mmoles) in methanol (20 ml) was made alkaline with 1 M sodium hydroxide solution (5.4 ml), then stirred for 30 minutes at room temperature, neutralized to pH 7.0, the solvent was distilled off, the aqueous residue was adjusted with 1 M hydrochloric acid to pH 3 and the liberated product was taken into ethyl acetate (3 x 100 ml). Combined organic

extracts were dried over anhydrous sodium sulfate, evaporated and the residue was crystallized from ethyl acetate - petroleum ether. The yield was 1.7 g (88%) of the product melting at 132-134°C. After recrystallization from the same solvent system the m.p. remained unchanged. $R_f$ 0.75/$S_1$, 0.75/$S_2$. $[\alpha]_D^{20}$ -23.2° (c 0.2, methanol). Composition: $C_{25}H_{38}N_4O_9$.

b) tert-Butyloxycarbonylalanyl-Nω-benzyloxycarbonyl-lysyl-seryl-glutaminyl-glycyl-glycyl-seryl-asparaginyl-phenylalanine amide

A solution of the preceding serine derivative (0.8 g, 1.49 mmoles), N-hydroxybenzotriazole (0.2 g) and N,N'-dicyclohexylcarbodiimide (0.3 g) in dimethylformamide (30 ml) was stirred and cooled at -10°C for 10 minutes. A precooled (-10°C) solution of glutaminyl-glycyl-glycyl-seryl-asparaginyl-phenylalanine amide (1.0 g, 1.51 mmoles) in the same solvent (50 ml) was added, the reaction mixture was stirred for 2 hours at 0°C and then allowed to stand for 8 hours at room temperature. After filtering off the formed N,N'-dicyclohexylurea the solution was evaporated and the noncrystalline residue was mixed with saturated brine (sodium chloride solution, 3.0 ml). After 1 hour of standing at 0°C the crystalline product was separated, washed and crystallized from 1-butanol saturated with water at 20°C by the addition of ethyl acetate to give 0.4 g (23%) of the substance melting at 225-227°C. A sample was recrystallized similarly and the m.p. was unchanged. $R_f$ 0.25/$S_1$, 0.76/$S_2$. $[\alpha]_D^{20}$ -39.2° (c 0.1, 50% acetic acid). Amino-acid analysis: Ala 1.03, Lys 0.98, Ser 1.77, Glu 0.94, Gly 2.03, Asp 1.00, Phe 1.03. Composition: $C_{50}H_{73}N_{13}O_{17} \cdot H_2O$.

c) Alanyl-lysyl-seryl-glutaminyl-glycyl-glycyl-seryl-asparaginyl-phenylalanine amide

The preceding peptide derivative (0.4 g, 0.35 mmole) was de-protected by hydrogenation over Pd black (approx

20 mg) in 10% acetic acid (100 ml) at atmospheric pressure and room temperature for 2 hours. After removal of the catalyst the solution was evaporated, the residue was dried in a desiccator over sodium hydroxide and phosphorus pentoxide for 5 hours and dissolved in trifluoroacetic acid (1.8 ml). The solution was stirred for 1 hour at room temperature, the product was precipitated with ether, separated, washed, dried as above for 2 hours and dissolved in 50% methanol. A conversion on Zerolite (Trade Mark) FF anion exchanger in the acetate cycle (first batchwise, then on a column 1.8 x 25 cm) yielded the corresponding acetate. Combined methanolic eluates were evaporated, the residue was dissolved in 0.2 M acetic acid and purified by gel filtration on Sephadex G 15 as described in Example 1. The yield was 0.2 g (57%) of a uniform product. $R_f$ 0.02/$S_1$, 0.45/$S_2$. $[\alpha]_D^{20}$ -40.4$^o$ (c 0.1, 0.1 M acetic acid). Amino-acid analysis: Ala 1.09, Lys 1.03, Ser 1.65, Glu 1.00, Gly 1.97, Asp 1.02, Phe 0.98. Composition: $C_{37}H_{59}N_{13}O_{13}$ . 2 AcOH.

Example 4

a) Alanyl-Nω-benzyloxycarbonyllysyl-seryl-glutaminyl-glycyl-glycyl-seryl-asparaginyl-phenylalanine amide trifluoroacetate

A solution of the corresponding tert-butyloxycarbonyl-alanyl peptide amide (1.0 g, 0.87 mmole) and anisole (0.3 ml) in trifluoroacetic acid (2.7 ml) was stirred for 30 minutes at room temperature. The product was precipitated by the addition of ether, separated, washed and dried over sodium hydroxide and phosphorus pentoxide to give 0.9 g (89%) of the substance having $R_f$ 0.21/$S_1$, 0.65/$S_2$.

b) Benzyloxycarbonylglutaminyl-alanyl-N -benzyloxy-carbonyllysyl-seryl-glutaminyl-glycyl-glycyl-seryl-asparaginyl-phenylalanine amide

Benzyloxycarbonylglutamine (250 mg, 0.89 mmole),

N-hydroxybenzotriazole (120 mg) and N,N'-dicyclohexylcarbodiimide (200 mg) dissolved in dimethylformamide (30 ml) were stirred and cooled (-10$^{o}$C) for 10 minutes. A precooled solution (10$^{o}$C) of the preceding amide liberated from its trifluoroacetate (995 mg; 0.87 mmole) by the addition of 0.15 ml of N-ethylpiperidine in dimethylformamide (30 ml) was added. The reaction mixture was stirred for 2 hours at 0$^{o}$C and then allowed to stand for 8 hours at room temperature. After evaporation to approx a half of the initial volume and subsequent cooling (1 hour at 0$^{o}$C) the N,N'-dicyclohexylurea precipitate was filtered off and the filtrate was evaporated to dryness. The solid residue was mixed with water (3 ml) and the crystalline product was separated and crystallized from acetic acid - ethyl acetate to give 200 mg (18%) of the substance melting at 251-254$^{o}$C (unchanged after recrystallization. $R_f$ 0.02/$S_1$, 0.03/$S_2$. $[\alpha]_D^{20}$ -38.8$^{o}$ (c 0.1, 25% acetic acid). Amino-acid analysis: Glu 1.84, Ala 0.95, Lys 1.11, Ser 1.73, Gly 2.04, Asp 1.00, Phe 1.09. Composition: $C_{58}H_{79}N_{15}O_{19}$. 2 $H_2O$.

c) Glutaminyl-alanyl-lysyl-seryl-glutaminyl-glycyl-glycyl-seryl-asparaginyl-phenylalanine alanine amide

The preceding benzyloxycarbonylglutaminyl peptide amide (100 mg, 0.08 mmole) was hydrogenated in 10% acetic acid (100 ml) acidified with 1 M hydrochloric acid (0.4 ml) over Pd black (approx 30 mg) at atmospheric pressure and room temperature for 3 hours. After removal of the catalyst the filatrate evaporated, the residue was dissolved in water (5 ml) and again evaporated; this was repeated twice. The solid residue was dissolved in 0.2 M acetic acid and purified by gel filtration on Sephadex G 15 as described in Example 1. Yield 70 mg (75%). $R_f$ 0.00/$S_1$, 0.25/$S_2$, $[\alpha]_D^{20}$ -47.8$^{o}$ (c 0.1 M acetic acid). Amino-acid analysis: Glu 1.92, Ala 1.00, Lys 0.99, Ser 1.77, gly 2.04, Asp 1.01, Phe 0.97. Composition: $G_{42}H_{67}N_{15}O_{15}$ . 2 HC1 . 4 $H_2O$.

Example 5

a) Benzyloxycarbonylasparaginyl-phenylalanine 2-phenylethylamide was prepared by the mixed-anhydride method technique as was used for the corresponding methyl ester in Example 1. Yield 70%, m.p. 239-243°C (acetic acid - ether). Composition: $C_{29}H_{32}N_4O_5$ . 0.5 AcOH. The product was de-protected by the common technique with the use of a hydrogen bromide solution in acetic acid. After 1 hour of standing at room temperature the hydrobromide was precipitated by the addition of ether, separated, thoroughly washed with the same solvent and dried for 12 hours over sodium hydroxide and phosphorus pentoxide to yield asparaginyl-phenylalanine 2-phenylethylamide hydrobromide, $R_f$ 0.48/$S_1$, 0.53/$S_2$.

b) Benzyloxycarbonylglycyl-glycyl-serine hydrazide

A solution of benzyloxycarbonylglycyl-glycine (13.31 g, 50 mmoles), N-hydroxysuccinimide (5.75 g) and N,N'-dicyclohexylcarbodiimide (11.3 g) in dimethylformamide (180 ml) was stirred and cooled (-10°C) for 1 hour. A precooled (-10°C) solution of serine methyl ester, liberated from its hydrochloride (7.8 g, 50 mmoles) with N-ethylpiperidine (6.85 ml), in the same solvent (50 ml) was added, and the reaction mixture was stirred for 3 hours at 0°C and allowed to stand for 8 hours at room temperature; the N,N'-dicyclohexylurea precipitate was filtered off and the filtrate was admixed with 100% hydrazine hydrate (15 ml). After 3 days of standing at room temperature the formed hydrazide was separated, washed with ethanol and crystallized from aqueous ethanol. Yield 15.2 g (83%), m.p. 172-175°C (unchanged after recrystallization). Composition: $C_{15}H_{21}N_5O_6$.

c) Benzyloxycarbonylglycyl-glycyl-seryl-asparaginyl-phenylalanine 2-phenylethylamide

A solution of the preceding hydrazide (3.7 g, 10 mmoles) and concentrated hydrochloric acid (4 ml) in

dimethylformamide (80 ml) was precooled to -30°C and treated with a solution of sodium nitrate (690 mg) in water (2.8 ml). After 10 minutes of stirring at -25°C the mixture was adjusted with N-ethylpiperidine to pH 6.9 and added to a precooled (-30°C) solution of asparaginyl-phenylalanine 2-phenylethyl amide, liberated from the preceding hydrobromide (10 mmoles) with approx 1.6 ml of N-ethylpiperidine, in dimethylformamide (50 ml). After standing at 3°C the reaction mixture was evaporated, the product was precipitated by the addition of water, separated, triturated successively with 1 M hydrochloric acid, water, 5% sodium hydrogencarbonate and water and crystallized from acetic acid - ether. The yield was 2.45 g (43%), m.p. 225-229°C.

d) Glycyl-glycyl-seryl-asparaginyl-phenylalanine 2-phenylamide was obtained by de-protecting the previous benzyloxycarbonyl derivative with hydrogen bromide in acetic acid to give the hydrobromide, m.p. 227-230°C, $R_f$ 0.52/$S_1$, 0.71/$S_2$, composition: $C_{28}H_{37}N_7O_7$ . 2 HBr . 0.5 AcOH. The title base was liberated from the hydrobromide with 10% aqueous ammonia in dimethyl formamide, isolated by dilution with water and crystallized from dimethylformamide - ether. Yield 51%, m.p. 223-226°C. $R_f$ 0.61/$S_1$, 0.74/$S_2$.

Example 6

Pyroglutamyl-glycyl-glycyl-seryl-asparaginyl-phenylalanine 2-phenylethylamide was prepared from pyroglutamic acid and glycyl-glycyl-seryl-asparaginyl-phenylalanine 2-phenylethylamide using the carbodiimide method similarly as described in the preceding Example 5. Yield 72%, m.p. 209-211°C, $R_f$ 0.25/$S_1$, 0.67/$S_2$.

Example 7

a) Benzyloxycarbonylseryl-asparaginyl-glycine 4,4'-dimethoxybenzhydrylamide was obtained from benzyloxycarbonylseryl-asparagine hydrazide and glycine 4,4'-dimethoxybenz-

hydrylamide by the procedure described in Example 1. Yield 64%, m.p. 160-162°C (dimethylformamide - methanol - ether), $R_f$ 0.68/$S_1$, 0.80/$S_2$.

b) Nα-Benzyloxycarbonylglutaminyl-glycyl-glycyl-seryl-asparaginyl-glycine-4,4'-dimethoxybenzhydrylamide, similarly from Nα-benzyloxycarbonylglutaminyl-glycyl-glycine hydrazide and seryl-asparaginyl-glycine 4,4'-dimethoxybenzhydrylamide, yield 54%, m.p. 200-202 C (dimethylformamide - ethyl acetate), $R_f$ 0.38/$S_1$, 0.85/$S_2$, composition: $C_{41}H_{51}N_9O_{13} \cdot H_2O$.

c) Pyroglutamyl-glycyl-glycyl-seryl-asparaginyl-glycine amide was prepared by catalytic hydrogenation of the preceding compound in 10% acetic acid acidified with hydrochloric acid as described in Example 1 and subsequent de-protection of the 4,4'-dimethoxybenzhydrylamide base ($R_f$ 0.10/$S_1$, 0.63/$S_2$, 0.36/$S_3$) with trifluoroacetic acid in boiling anisole. Yield 66% (benzhydrylamide cleavage step), $R_f$ 0.01/$S_1$, 0.03/$S_2$, $[\ ]_D^{20}$ -27.5° (c 0.1, 0.1 M acetic acid).

With the use of analogous preparative procedures, the following peptide compounds of the invention were also prepared:

Glutaminyl-glycyl-glycyl-seryl-asparaginyl-phenylalanine 2-phenylethylamide, $R_f$ 0.18/$S_1$, 0.51/$S_2$.

Glutaminyl-glycyl-glycyl-seryl-asparaginyl-phenylalanyl-phenylalanine amide, $R_f$ 0.08/$S_1$, 0.31/$S_2$.

Pyroglutamyl-glycyl-glycyl-seryl-asparaginyl-phenylalanyl-phenylalanine amide, m.p. 250-253°C (dimethylformamide - ether), $R_f$ 0.22/$S_1$, 0.51/$S_2$.

Glutaminyl-glycyl-glycyl-seryl-asparaginyl-alanine amide, $R_f$ 0.00/$S_1$, 0.11/$S_2$.

Glutaminyl-glycyl-glycyl-seryl-asparaginyl-alanyl-alanine amide, m.p. 181-185°C (decompn), $R_f$ 0.00/$S_1$, 0.04/$S_2$.

Alanyl-lysyl-seryl-glutaminyl-glycyl-glycyl-seryl-asparaginyl-alanine amide hydrogen-trifluoroacetate, m.p. 178-181°C (decompn), $R_f$ 0.00/$S_1$, 0.02/$S_2$.
Alanyl-lysyl-seryl-glutaminyl-glycyl-glycyl-seryl-asparaginyl-glycine amide, $R_f$ 0.01/$S_1$, 0.18/$S_2$.

## Claims

1. Serum thymic factor peptide analogs of the general formula I

A - Gly - Gly - Ser - Asn - B - C - NH - R (I)

in which A is pGlu, Gln, Ala-Lys-Ser, pGlu-Ala-Lys-Ser or Gln-Ala-Lys-Ser, B and C are Gly, Phe, Leu, Ala or a direct bond and R is H, an alkyl with 1 to 6 carbon atoms or a 2-phenylethyl group.

2. Alanyl-lysyl-seryl-glutaminyl-glycyl-glycyl-seryl-asparagine.

3. Alanyl-lysyl-seryl-glutaminyl-glycyl-glycyl-seryl-asparaginyl-glycine amide.

4. Alanyl-lysyl-seryl-glutaminyl-glycyl-glycyl-seryl-asparaginyl-phenylalanine amide.

5. Pyroglutamyl-alanyl-lysyl-seryl-glutaminyl-glycyl-glycyl-seryl-asparaginyl-glycine amide.

6. Pyroglutamyl-alanyl-lysyl-seryl-glutaminyl-glycyl-glycyl-seryl-asparaginyl-phenylalanine amide.

7. Pyroglutamyl-glycyl-glycyl-seryl-asparaginyl-glycine amide.

8. Pyroglutamyl-glycyl-glycyl-seryl-asparaginyl-phenylalanine amide.

9. Alanyl-lysyl-seryl-glutaminyl-glycyl-glycyl-seryl-asparaginyl-phenylalanine 2-phenylethylamide.

10. Pyroglutamyl-glycyl-glycyl-seryl-asparaginyl-phenylalanine 2-phenylethylamide.

11. Glutaminyl-glycyl-glycyl-seryl-asparaginyl-phenylalanine amide.

Claims (Austria)

1. A process for the preparation of serum thymic factor peptide analogs of the general formula I

A - Gly - Gly - Ser - Asn - B - C - NH - R (I)

in which A is pGlu, Gln, Ala-Lys-Ser, pGlu-Ala-Lys-Ser or Gln-Ala-Lys-Ser, B and C are Gly, Phe, Leu, Ala or a direct bond and R is H, and alkyl with 1 to 6 carbon atoms or a 2-phenylethyl group, which comprises reacting a peptide compound of the general formula II

Gly - Gly - Ser - Asn - B - C - NH - R (II)

in which B, C and R have the same meanings as in formula I, with a compound of the general formula III

X - A (Y) (III)

in which A has the same meaning as in formula I and X and Y are protective groups for protecting alpha or omega amino groups, with the proviso that X and Y can also be H atoms when A is pGlu, to yield a peptide compound of the general formula IV

X - A (Y) - Gly - Gly - Ser - Asn - B - C - NH - R (IV)

in which A, B, C and R have the same meanings as in formula I and X and Y have the same meanings as in formula III, with an optional subsequent step involving successive or simultaneous removal of the protective groups.

2. A process according to claim 1 for the preparation of serum thymic factor peptide analogs of the general formula I, in which A is pGlu and B, C and R have the same meanings as defined in claim 1, which comprises reacting a peptide compound of the general formula II, in which B, C and R have the above meaning, with pyroglutamic acid.

3. Serum thymic factor peptide analogs whenever prepared by a process substantially in accordance with either of the preceding claims or an obvious equivalent thereof.